# EUROPEAN PATENT APPLICATION

(11) **EP 4 212 626 A1**
(43) Date of publication of application: **19.07.2023**
(21) Application number: 21866837.4
(22) Date of filing: 09.09.2021
(51) Int. Cl.: C12P 21/02, A23C 11/10, A23J 3/16, A23L 11/00, A23L 11/45, A23L 11/60, A23L 11/65

(54) **METHOD OF PRODUCING PROCESSED PROTEIN**

(30) Priority: 10.09.2020 JP 2020152014
(71) Applicant: Amano Enzyme Inc., Nagoya-shi Aichi 460-8630 (JP)
(72) Inventor: SAKAI, Kiyota, Kakamigahara-shi, Gifu 509-0109 (JP)
(74) Representative: Müller-Boré & Partner Patentanwälte PartG mbB
(86) International application number: PCT/JP2021/033199
(87) International publication number: WO 2022/054880

(57) **Abstract**

The purpose of the present invention is to provide a processing technology for enhancing the crosslinkage effect of a protein. A processed protein obtained by a method of producing a processed protein, said method comprising a crosslinkage step for treating a protein with laccase and transglutaminase, has an enhanced crosslinkage effect.

## Description

### TECHNICAL FIELD

The present invention relates to a method of producing a processed protein. More specifically, the present invention relates to a processing technique for enhancing the crosslinking effect of a protein.

### BACKGROUND ART

A protein is crosslinked for a predetermined purpose. For example, for the purpose of improving an existing processed food product, creating a processed food product having new preference characteristics, and the like, a crosslinking treatment is performed on a protein as a food product material.

As a means used for crosslinking a protein, a transglutaminase is known. The transglutaminase crosslinks a side chain carbamoyl group of a glutamine residue and a side chain amino group of a lysine residue of a protein with an isopeptide bond (Non-Patent Document 1). For example, Patent Documents 1 and 2 describe that the strength of tofu is enhanced by using a transglutaminase in production of tofu.

Patent Document 3 describes that a protein is crosslinked by a laccase, and further describes that a laccase crosslinks a protein by forming an ε-amino group of lysine with another amino group to form a Schiff base.

### PRIOR ART DOCUMENTS

### PATENT DOCUMENTS

Patent Document 1: Japanese Patent Laid-open Publication No. 2007-312723
Patent Document 2: Japanese Patent Laid-open Publication No. 2015-180197
Patent Document 3: Japanese Patent Laid-open Publication No. H11-276162

### NON-PATENT DOCUMENT

Non-Patent Document 1: SEIKAGAKU (Biochemistry), Vol. 81, No. 8, p. 708-711, 2009

### SUMMARY OF THE INVENTION

### PROBLEMS TO BE SOLVED BY THE INVENTION

As a method for crosslinking a protein, a method using a predetermined enzyme is known as described above, but a technique for further enhancing the crosslinking effect is desired so as to be able to cope with the increasing need for modification required for food products.

Therefore, an object of the present invention is to provide a processing technique for enhancing the crosslinking effect of a protein.

### MEANS FOR SOLVING THE PROBLEM

The present inventor has found that the crosslinking effect of a protein is remarkably enhanced by combining a laccase and a transglutaminase. In view of the fact that, even when a protein is crosslinked by changing the use amount of a laccase, the crosslinking effect to be obtained is generally poor and that both of a laccase and a transglutaminase has the same side chain amino acid of lysine as a substrate, it has been unexpected that the crosslinking effect of the protein is remarkably enhanced by a combination of these enzymes. The unexpected effect obtained by such a combination has been remarkable to the extent that the crosslinking effect of the protein is remarkably enhanced by further combining a transglutaminase even when a large amount of laccase is used so that the crosslinking effect of the protein is almost saturated. The present inventor has conducted further studies based on the findings, leading to the completion of the present invention. That is, the present invention provides inventions of the following aspects.

Item 1. A method of producing a processed protein, including a crosslinking step of causing a laccase and a transglutaminase to act on a protein.

Item 2. The method of producing a processed protein described in item 1, in which the protein is soybean protein.

Item 3. The method of producing a processed protein described in item 1 or 2, in which the processed protein is tofu.

Item 4. The method of producing a processed protein described in item 1 or 2, in which the processed protein is fermented soy milk.

Item 5. The method of producing a processed protein described in any one of items 1 to 4, in which the laccase is used in an amount of 0.02 U or more per 1 g of the protein.

Item 6. The method of producing a processed protein described in any one of items 1 to 5, in which the laccase is used in an amount of 30 U or more per 1 g of the protein.

Item 7. The method of producing a processed protein described in any one of items 1 to 5, in which the transglutaminase is used in an amount of 0.02 U or more per 1 g of the protein.

Item 8. The method of producing a processed protein described in any one of items 1 to 7, in which the transglutaminase is used in an amount of 0.0004 U or more per 1 U of the laccase.

Item 9. The method of producing a processed protein described in any one of items 1 to 8, in which the laccase is derived from Trametes hirsuta.

### ADVANTAGES OF THE INVENTION

According to the present invention, there is provided a processing technique for enhancing the crosslinking effect of a protein.

### BRIEF DESCRIPTION OF THE DRAWINGS

Fig. 1 shows the relationship between the presence or absence of a laccase treatment and a difference in concentration of a laccase in production of tofu, and the strength of tofu, as obtained in a preliminary test example.
Fig. 2 shows the relationship between the presence or absence of a laccase treatment, the presence or absence of a transglutaminase treatment, and a difference in concentration of a transglutaminase in production of tofu, and the strength of tofu, as obtained in Test Example 1.

### EMBODIMENTS OF THE INVENTION

A method of producing a processed protein of the present invention includes a crosslinking step of causing a laccase and a transglutaminase to act on a protein. Hereinafter, the method of producing a processed protein will be specifically described.

A supply source of the protein used in the present invention is not particularly limited, and a material containing a protein is used without particular limitation. Examples of the material containing a protein include materials used in various industrial fields such as food product materials, medical materials, and industrial materials. Specific examples of the protein include plant proteins and animal proteins. Examples of the plant proteins include bean proteins such as soybean protein and horse bean protein; and grain proteins such as wheat protein, rye protein, oat protein, and corn protein. Examples of the animal proteins include fish protein, livestock protein, egg protein, milk protein, and tendon protein (such as gelatin and collagen).

These proteins may be used singly or in combination of a plurality of kinds thereof. Among these proteins, from the viewpoint of further enhancing the crosslinking effect of the protein, plant proteins are preferable, bean proteins are more preferable, and soybean protein is particularly preferable.

As specific forms of these proteins, those obtained by preparing a material containing a material in an appropriate form (protein-containing material) are used. Specifically, the protein-containing material may be in a form in which a protein and an enzyme are efficiently in contact with each other, and preferably, in the case of an animal protein, examples of the form thereof include minced meat or ground meat of animal food materials (such as fish meat and livestock meat); egg liquids such as whole liquid egg, egg white liquid, and egg yolk liquid; and animal milk such as cow milk and goat milk; and in the case of a plant protein, examples of the form thereof include plant milk, typically, squeeze (milk) of water-absorbed products of food materials (beans such as soybeans and horse beans; grains such as wheat, rye, oats, corn; and the like), and bean powder and grain powder. Regarding the tendon protein (such as gelatin and collagen), a purified protein can be used as the protein-containing material.

These protein-containing materials may be used singly or in combination of a plurality of kinds thereof. Among these protein-containing materials, from the viewpoint of further enhancing the crosslinking effect of the protein, plant milk is preferable and soy milk is more preferably preferable.

The amount of the protein in the plant milk is not particularly limited, and is, for example, 3 g/100 mL or more, preferably 3.8 g/100 mL or more, more preferably 4.2 g/100 mL or more, further preferably 4.8 g/100 mL or more, and particularly preferably 5 g/100 mL or more. The upper limit of the protein amount range in the plant milk is not particularly limited, and is, for example, 8 g/100 mL or less. The material solid content of the plant milk (for example, the soybean solid content in the case of soy milk) is not particularly limited, and is, for example, 6 wt% or more, preferably 8 wt% or more, more preferably 9 wt% or more, further preferably 9.5 wt% or more, and particularly preferably 10 wt% or more. The upper limit of the material solid content range of the plant milk is not particularly limited, and is, for example, 18 wt% or less or 16 wt% or less.

The protein-containing material may contain a quality improving agent such as an emulsifier (such as sucrose fatty acid ester, phospholipid, monoglyceric fatty acid ester, organic acid monoglyceric fatty acid ester, sorbitan fatty acid ester, polyoxyethylene sorbitan fatty acid ester, polyglycerin fatty acid ester, or propylene glycol fatty acid ester) and a pH adjusting agent (such as sodium carbonate, sodium hydrogen carbonate, or calcium carbonate), a coloring agent, a flavor, a seasoning (such as dietary salt or sugar (sucrose)), or the like according to the form of a target processed protein, and the like, as long as the effect of the present invention is not impaired.

The laccase used in the present invention is an enzyme having phenol oxidase activity (EC1.10.3.2). Specific examples of the laccase include laccases derived from microorganisms such as fungi and bacteria, and more specific examples thereof include laccases derived from the genera Aspergillus, Neurospora, Podospora, Botrytis, Collybia, Fomes, Lentinus, Pleurotus, Pycnoporus, Pyricularia, Trametes, Rhizoctonia, Rigidoporus, Coprinus, Psatyrella, Myceliophtera, Schtalidium, Polyporus, Phlebia, Coriolus, and the like.

These laccases may be used singly or in combination of a plurality of kinds thereof. Among these laccases, a laccase derived from the genus Trametes is preferable, and a laccase derived from Trametes hirsuta is particularly preferable, from the viewpoint of further enhancing the crosslinking effect of the protein.

The use amount of the laccase is not particularly limited, but the use amount of the laccase per 1 g of the protein is, for example, 0.02 U or more. From the viewpoint of further enhancing the crosslinking effect of the protein, the use amount of the laccase per 1 g of the protein is preferably 0.2 U or more, more preferably 2 U or more, further preferably 10 U or more, even more preferably 30 U or more, and further more preferably 45 U or more. The upper limit of the use amount range of the laccase is not particularly limited, but the use amount of the laccase per 1 g of the protein is, for example, 500 U or less, 200 U or less, 100 U or less, 80 U or less, 60 U or less, or 55 U or less.

As for the laccase activity, when a 0.1 ml enzyme liquid is added to 3 ml of a 1.0 mg/ml solution of 2,2'-Azino-di-[3-ethylbenzthiazoline sulfonate] (ABTS) as a substrate at 25°C, the amount of the enzyme that increases the absorbance at 405 nm by 1.0 OD per minute is defined as 1 unit.

The transglutaminase used in the present invention is an enzyme having transglutaminase activity (EC2.3.2.13). As the transglutaminase, both a calcium-dependent transglutaminase, which requires calcium for active expression, and a calcium-independent transglutaminase, which does not require calcium for active expression, are mentioned. Specific examples of the transglutaminase include transglutaminases derived from microorganisms, mammals, fish, and the like, and more specific examples thereof include transglutaminases derived from microorganisms belonging to the genera Streptomyces, Bacillus, Geobacillus, and the like; mammals such as guinea pig (liver), cow (blood), and pig (blood); and fish such as salmon, red sea bream, and cod.

These transglutaminases may be used singly or in combination of a plurality of kinds thereof. Among the calcium-dependent and calcium-independent transglutaminases, from the viewpoint of further enhancing the crosslinking effect of the protein, a calcium-independent transglutaminase is preferable. Among the transglutaminases, from the viewpoint of further enhancing the crosslinking effect of the protein, a microorganism-derived transglutaminase is preferable, a transglutaminase derived from the genus Streptomyces is more preferable, and a transglutaminase derived from Streptomyces mobaraensis is particularly preferable.

The use amount of the transglutaminase is not particularly limited, but the use amount of the transglutaminase per 1 g of the protein is, for example, 0.001 U or more, 0.01 U or more, or 0.02 U or more. From the viewpoint of further enhancing the crosslinking effect of the protein, the use amount of the transglutaminase per 1 g of the protein is preferably 0.2 U or more, more preferably 0.5 U or more, further preferably 1 U or more, even more preferably 1.5 U or more, and further more preferably 2 U or more, 5 U or more, 10 U or more, or 15 U or more. The upper limit of the use amount range of the transglutaminase is not particularly limited, but the use amount of the transglutaminase per 1 g of the protein is, for example, 200 U or less, 100 U or less, 50 U or less, 30 U or less, 25 U or less, or 20 U or less.

As for the transglutaminase activity, when benzyloxycarbonyl-L-glutaminylglycine and hydroxylamine are reacted at 37°C as substrates, the amount of the enzyme that produces 1 µmol of hydroxamic acid per minute is defined as 1 unit.

The ratio of the use amounts of the laccase and the transglutaminase is determined depending on the use amount of each enzyme, but from the viewpoint of further enhancing the crosslinking effect of the protein, the use amount of the transglutaminase with respect to 1 U of the laccase is preferably 0.00001 U or more, 0.0001 U or more, or 0.0004 U or more, more preferably 0.004 U or more, further preferably 0.01 U or more, even more preferably 0.02 U or more, further more preferably 0.03 U or more, and particularly preferably 0.04 U or more, 0.1 U or more, 0.2 U or more, or 0.3 U or more. The upper limit of the use amount range of the transglutaminase with respect to 1 U of the laccase is not particularly limited, and is, for example, 10 U or less, 5 U or less, 2 U or less, 1 U or less, 0.6 U or less, 0.5 U or less, or 0.4 U or less.

In the crosslinking step, a protein composition containing a protein-containing material, a laccase, and a transglutaminase is prepared by mixing a protein-containing material, a laccase, and a transglutaminase, typically, adding a laccase and a transglutaminase to a protein-containing material, and the protein composition is maintained in a heated state, thereby allowing the crosslinking of the protein by the laccase and the transglutaminase to proceed.

The temperature of the protein-containing material at the time of preparing the protein composition is not particularly limited, and the material may be in a non-heated state or in a heated state, but is preferably in a heated state. The temperature when the material is in a heated state is not particularly limited, but is preferably a temperature for allowing crosslinking described below to proceed.

In the crosslinking step, in addition to the crosslinking of the protein, a treatment according to the form of a target processed protein may be simultaneously performed. For example, when the form of the processed protein is tofu, a coagulation reaction may be performed simultaneously, and when the form of the processed protein is a fermented product such as fermented soy milk (yogurt such as soy milk yogurt, and the like), fermentation may be performed simultaneously.

Therefore, the protein composition to be subjected to the crosslinking step can contain other materials necessary for obtaining the form of a target processed protein, in addition to the protein-containing material, the laccase, and the transglutaminase. Examples of the other materials include coagulants used for the form of tofu, and fermenting bacteria (such as yogurt inoculums) used for the form of a fermented product such as fermented soy milk (yogurt such as soy milk yogurt, and the like).

The coagulant is not particularly limited, and a coagulant generally used in production of tofu can be used. Specifically, examples of the coagulant include a salt coagulant and an acid coagulant. Examples of the salt coagulant include magnesium chloride, magnesium sulfate, calcium sulfate, and calcium chloride, and examples of the acid coagulant include glucono delta-lactone.

These coagulants may be used singly or in combination of a plurality of kinds thereof. Among these, from the viewpoint of further enhancing the crosslinking effect of the protein, the coagulant is preferably a salt coagulant and more preferably magnesium chloride.

The use amount of the coagulant is not particularly limited, and is, for example, 0.1 wt% or more, 0.2 wt% or more, 0.3 wt% or more, or 0.5 wt% or more. From the viewpoint of further enhancing the crosslinking effect of the protein, the use amount of the coagulant is preferably 0.75 wt% or more, more preferably 1 wt% or more, further preferably 1.5 wt% or more, and even more preferably 2 wt% or more. The upper limit of the use amount range of the coagulant is not particularly limited, and is, for example, 4 wt% or less or 3 wt% or less.

The fermenting bacteria such as yogurt inoculums are not particularly limited, and can be appropriately determined in consideration of an optimum temperature of a laccase and a transglutaminase, and the like. Examples of the yogurt inoculums include thermophilic bacterial species having an optimum temperature for growth of about 37°C to 45°C and mesophilic bacterial species having an optimum temperature for growth of 20°C to 30°C, and any bacterial species can be used. That is, even when the crosslinking step is performed under heating conditions, not only thermophilic bacterial species but also mesophilic bacterial species can be used. Examples of the thermophilic bacterial species include bacterial species belonging to the genera Streptococcus, Enterococcus, and the like. Examples of the mesophilic bacterial species include bacterial species belonging to the genera Lactococcus (preferably Lactococcus lactis subsp. cremoris), Lactobacillus, Acetobacter (preferably Acetobacter orientalis), and the like.

These yogurt inoculums may be used singly or in combination of two or more kinds thereof. Among these yogurt inoculums, bacterial species belonging to the genus Lactococcus and bacterial species belonging to the genera Lactobacillus and Acetobacter are preferable, Lactococcus lactis subsp. cremoris and Acetobacter orientalis are more preferable, and a combination of these yogurt inoculums is further preferable.

The temperature at which the protein composition is provided for allowing crosslinking to proceed can be appropriately determined depending on an optimum temperature of a laccase and a transglutaminase, the form of a target processed protein, and the like, and is, for example, 35 to 70°C, preferably 38 to 60°C, and more preferably 40 to 57°C. More specifically, when the form of a target processed protein is tofu, the temperature for allowing crosslinking to proceed can be appropriately determined depending on an optimum temperature of a laccase and a transglutaminase, and the like, and is, for example, 50 to 70°C, preferably 52 to 60°C, and more preferably 54 to 57°C. When the form of a target processed protein is a fermented product such as fermented soy milk (yogurt such as soy milk yogurt, and the like), the temperature for allowing crosslinking to proceed can be appropriately determined depending on an optimum temperature of a laccase and a transglutaminase, an optimum temperature for growth of fermenting bacteria (such as yogurt bacterial species), and the like, and is, for example, 35 to 50°C, preferably 38 to 46°C, and more preferably 40 to 44°C.

The time for crosslinking is not particularly limited, and is, for example, 30 minutes or longer and preferably 1 hour or longer, although it depends on a treatment to be simultaneously performed in the crosslinking step (for example, a coagulation treatment when the form of a processed protein is tofu, a fermentation treatment when the form of a processed protein is a fermented product such as fermented soy milk, and the like), the scale of the protein composition, and the like. The upper limit of the time range for crosslinking is not particularly limited, and is, for example, 30 hours or shorter, 24 hours or shorter, 12 hours or shorter, 8 hours or shorter, or 4 hours or shorter.

After completion of the crosslinking reaction, an arbitrary treatment suitable for the form of a processed protein can be appropriately performed. Examples of the arbitrary treatment include a boiling step, a firing (roasting, toasting, baking, grilling, broiling) step, a steaming step, and a frying step. These steps may be used singly or in combination of a plurality of kinds thereof.

A specific form of a processed protein (that is, a crosslinked protein) obtained by the production method of the present invention is determined according to the type of the protein, the type of the protein-containing material, and the crosslinking effect to be obtained, and examples thereof include tofu such as cotton tofu, silken tofu, and packed tofu; fermented products such as fermented soy milk and fermented animal milk (yogurt such as soy milk yogurt and animal milk yogurt, cheese, and the like); other soybean processed products such as soybean meat, deep-fried tofu, and tofu hamburger steak; fish paste products such as fish balls, fish rings, minced and steamed fish, fish sausages, and minced fish; livestock processed products such as hamburger steak, sausage, meat dumpling, and minced meat cutlet; egg processed products such as rolled egg, egg filling, scrambled egg, tinned egg, omelet sheet, and long egg; grain processed products such as noodles, confectionery, and bakery; viscoelastic confectionery such as jelly, gummies, and chewing candies; and food product adhesives.

Among these processed proteins, from the viewpoint of enjoying a further enhanced crosslinking effect, tofu, fermented soy milk (preferably soy milk yogurt), and other soybean processed products are preferable, tofu and other soybean processed products are more preferable, and tofu is further preferable.

The crosslinking effect to be obtained of a processed protein by the production method of the present invention is not particularly limited as long as it has a characteristic of being changed by crosslinking of the protein, and examples thereof include improvement of organization characteristics such as enhancement of compressive strength, enhancement of viscoelasticity, and improvement of gel forming ability.

### EXAMPLES

Hereinafter, the present invention will be specifically described by means of Examples; however, the present invention is not to be construed as being limited to the following Examples.

### (1) Enzyme Activity Value Measurement Method

### (1-1) Laccase Activity Value Measurement Method

In the following Test Examples, the enzyme activity of the laccase was measured by the method described below using 2,2'-Azino-di-[3-ethylbenzthiazoline sulfonate] (ABTS, manufactured by Boehringer Mannheim) as a substrate.

ABTS was dissolved in a 25 mM citrate buffer solution (pH 3.2) at a concentration of 1.0 mg/ml to prepare a substrate solution. In a cuvette, 3.0 ml of this substrate solution was placed and preheated at 25°C, a 0.1 ml enzyme liquid was then added, stirred, and incubated at 25°C, and the absorbance at 405 nm after 1 minute and 3 minutes was measured. The amount of the enzyme that increased the absorbance at 405 nm by 1.0 OD per minute under this condition was defined as 1 unit (U).

### (1-2) Transglutaminase Activity Value Measurement Method

In the following Test Examples, the enzyme activity of the transglutaminase was measured by the method described below using benzyloxycarbonyl-L-glutaminylglycine and hydroxylamine as substrates and using the following substrate solution and coloring solution.

### (Substrate Solution)

A substrate solution was prepared by dissolving 2.42 g of 2-amino-2-hydroxymethyl-1.3-propanediol, 0.70 g of hydroxyammonium hydrochloride, 0.31 g of reduced glutathione, and 1.01 g of Z-Gln-Gly (benzyloxycarbonyl-L-glutaminylglycine) in distilled water to make a total amount of 100 mL (pH 6.0).

### (Coloring Solution)

A coloring solution was prepared by mixing 30 mL of a 3 M hydrochloric acid solution, 30 mL of a 12 wt% trichloroacetic acid solution, and 30 mL of 5 wt% iron(III) chloride solution.

An enzyme was diluted with 200 mM of Tris-HCl (pH 6.0) to an appropriate concentration to prepare a sample solution. After 100 µL of the substrate solution was added to 10 µL of the sample solution and mixed, the mixture was reacted at 37°C for 10 minutes. After 100 µL of the coloring solution was added to stop the reaction and form an Fe complex, the absorbance at 525 nm was measured. As a control, the previously heat-inactivated sample solution was used and reacted in the same manner, the absorbance of the sample solution was measured, and the absorbance difference from the non-inactivated sample solution was determined. Separately, a calibration curve was created using L-glutamic acid-γ-monohydroxamic acid instead of the sample solution, and the amount of hydroxamic acid generated from the absorbance difference was determined. The enzyme activity of producing 1 µmol of hydroxamic acid per minute was defined as 1 unit (U).

### (2) Materials

Soy milk: "Organic Soy Milk" manufactured by Meiraku Group, soybean protein concentration: 5 g/100 mL, soybean solid content: 10 wt%
Laccase: Laccase derived from Trametes hirsuta
Transglutaminase: Transglutaminase derived from Streptomyces mobaraensis

### [Preliminary Test Example]

A soy milk composition was prepared by heating 20 mL of soy milk at 55°C for 5 minutes, adding 30 U, 60 U, or 120 U of a laccase aqueous solution per 1 g of the protein and mixing the mixture for 5 seconds or adding 0.6 mL (final concentration: 1.0 wt%) of a 10 wt% magnesium chloride aqueous solution, and mixing the mixture for 5 seconds, tofu was prepared by heating the soy milk composition at 55°C for 1 hour, and the obtained tofu was cooled at 4°C.

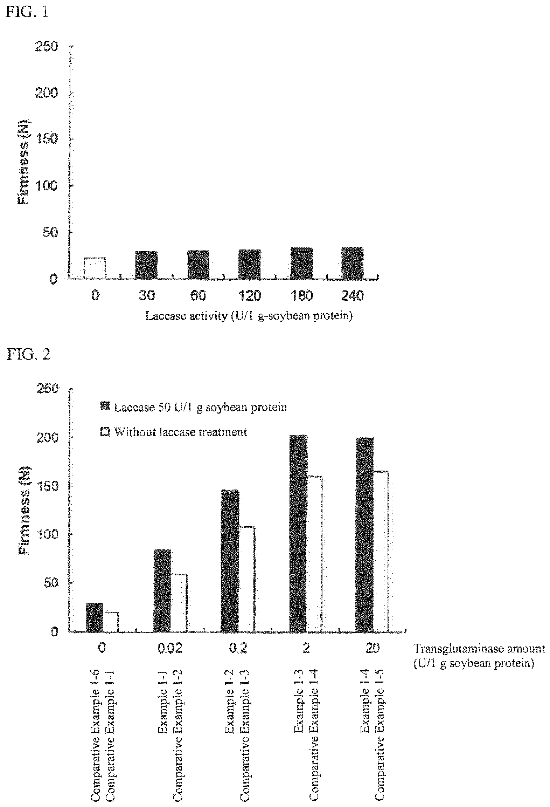

The strength (Firmness (N); specifically, compressive strength) of the obtained tofu was measured using a rheometer (COMPAC-100II SUN SCIENTIFIC CO., LTD.). The measurement conditions were as follows: Mode: 20, adapter: No. 13, repetition: 1 time, and pushing distance: 5 mm. Results are shown in Fig. 1.

As shown in Fig. 1, the strength of the tofu prepared using 30 U of a laccase was enhanced by 1.3 times, specifically, only 7 N, of the strength (22 N) of the tofu prepared with only a coagulant without using a laccase, and a slight enhancing effect of the tofu strength by the laccase was observed. On the other hand, even when the use amount of the laccase was further increased, the strength of the tofu was hardly enhanced, and the result that the substrate of the laccase was considered to be almost exhausted was shown.

### [Test Example 1]

A soy milk composition was prepared by heating 20 mL of soy milk at 55°C for 5 minutes, adding a laccase aqueous solution so as to be the amount shown in Table 1 per 1 g of the protein, mixing the mixture for 5 seconds, adding a transglutaminase aqueous solution so as to be the amount shown in Table 1 per 1 g of the protein, mixing the mixture for 5 seconds, adding 0.6 mL (final concentration: 1.0 wt%) of a 10 wt% magnesium chloride aqueous solution, and mixing the mixture for 5 seconds, tofu was prepared by heating the soy milk composition at 55°C for 1 hour (crosslinking step associated with coagulation), and the obtained tofu was cooled at 4°C (Comparative Examples 1-1 to 1-6 and Examples 1-1 to 1-4).

**[Table 1]**

| | Laccase use amount (U/1 g soybean protein) | Transglutaminase use amount (U/1 g soybean protein) |
|---|---|---|
| Comparative Example 1-1 | 0 | 0 |
| Comparative Example 1-2 | 0 | 0.02 |
| Comparative Example 1-3 | | 0.2 |
| Comparative Example 1-4 | | 2 |
| Comparative Example 1-5 | | 20 |
| Comparative Example 1-6 | 50 | 0 |
| Example 1-1 | 50 | 0.02 |
| Example 1-2 | | 0.2 |
| Example 1-3 | | 2 |
| Example 1-4 | | 20 |

The strength (Firmness (N); specifically, compressive strength) of the obtained tofu was measured using a rheometer (COMPAC-100II SUN SCIENTIFIC CO., LTD.). The measurement conditions were as follows: Mode: 20, adapter: No. 13, repetition: 1 time, and pushing distance: 5 mm. Results are shown in Fig. 2.

As shown in Fig. 2, the strength of the tofu of each of Comparative Examples 1-2 to 1-5 prepared by using only a transglutaminase treatment in combination with a coagulant was enhanced as compared with the tofu of Comparative Example 1-1 prepared with only a coagulant. Specifically, as compared with the strength (20 N) of the tofu of Comparative Example 1-1, the strength was enhanced to 3.0 times (specifically, increased by 39 N) when the addition amount of the transglutaminase per 1 g of soybean protein was 0.02 U (Comparative Example 1-2), the strength was enhanced to 5.4 times (specifically, increased by 88 N) when the addition amount of the transglutaminase per 1 g of soybean protein was 0.2 U (Comparative Example 1-3), the strength was enhanced to 8 times (specifically, increased by 140 N) when the addition amount of the transglutaminase per 1 g of soybean protein was 2 U (Comparative Example 1-4), and the strength was enhanced to 8.3 times (specifically, increased by 145 N) when the addition amount of the transglutaminase per 1 g of soybean protein was 20 U (Comparative Example 1-4).

On the other hand, in the preparation of Comparative Example 1-6 prepared by using only a laccase treatment in combination with a coagulant, the strength was enhanced to only 1.4 times (specifically, increased by 8 N) the strength (20 N) of the tofu of Comparative Example 1-1 although the laccase was used in an amount of 50 U per 1 g of soybean protein, similarly to the results shown in the preliminary test example described above.

On the other hand, as shown in the preliminary test example described above, when the laccase was used in an amount of 50 U per 1 g of soybean protein, it was considered that the substrate of the laccase (particularly, a side chain amino group of lysine) was almost exhausted; however, in the case of the tofu of each of Examples 1-1 to 1-4 prepared by using a combination of a laccase and a transglutaminase having the same substrate (a side chain amino group of a lysine residue) as that of the laccase, as compared with the strength (20 N) of the tofu of Comparative Example 1-1, the strength was enhanced to 4.2 times (specifically, increased by 64 N) in the case of combining 0.02 U of the transglutaminase per 1 g of soybean protein (Example 1-1), the strength was enhanced to 7.3 times (specifically, increased by 126 N) in the case of combining 0.2 U of the transglutaminase (Example 1-2), the strength was enhanced to 10 times (specifically, increased by 183 N) in the case of combining 2 U of the transglutaminase (Example 1-4), the strength was enhanced to 10 times (specifically, increased by 180 N) in the case of combining 20 U of the transglutaminase (Example 1-5), and an excellent strength enhancing effect exceeding the additive effect of the strength enhancing effect exhibited by each of the laccase and the transglutaminase alone was observed.

As is apparent from the comparison between Comparative Example 1-2, Comparative Example 1-3, and the like and Comparative Example 1-6, it is shown that the transglutaminase has a higher strength improving effect than the laccase. In particular, when the addition amount of the transglutaminase per 1 g of soybean protein is 2 U or more (Comparative Example 1-4 and Comparative Example 1-5), the strength improving effect is hardly observed, and it is considered that the substrate of the transglutaminase is used up. On the other hand, in the case of Examples 1-1 to 1-4 in which the laccase was used in combination with the transglutaminase, enhancement in strength of 37 N on average could be confirmed as compared with Comparative Examples 1-2 to 1-5 in which the transglutaminase was used singly, and even when Comparative Examples 1-4 and 1-5 and Examples 1-3 and 1-4 were compared, in Examples 1-3 and 1-4, enhancement in strength of 39 N on average could be confirmed as compared with Comparative Examples 1-4 and 1-5 in which the transglutaminase was used singly. In view of the fact that the amount of strength enhancement in Comparative Example 1-6 in which the laccase was used singly was only 8 N, from this viewpoint as well, it can be said that the tofu of each of Examples 1-1 to 1-4 had an excellent strength enhancing effect exceeding the additive effect of the strength enhancing effect.

The laccase and the transglutaminase have a common substrate (a side chain amino group of a lysine residue), and when the laccase and the transglutaminase coexist, it is reasonably expected that the reaction efficiency of the transglutaminase is reduced by the amount of the substrate lost by the reaction by the laccase. Although there is also a substrate for a laccase that is different from a transglutaminase (specifically, a tyrosine residue), since protein molecules to be enzymatically reacted are common between the laccase and the transglutaminase, a protein molecule in which tyrosine residues are crosslinked by the laccase reduces a contact opportunity between the lysine residue and the transglutaminase due to steric hindrance, and it is reasonably expected that the reaction efficiency by the transglutaminase is reduced also in this respect. In consideration of these points, when the laccase and the transglutaminase coexist, it is reasonably expected that the potential of each enzyme related to enhancement of the soybean protein cannot be completely exerted, and thus, even an additive effect cannot be expected, and furthermore, a synergistic effect cannot be expected even more. Therefore, as shown in Examples 1-1 to 1-4, it was unexpected that the tofu strength could be synergistically enhanced by using the laccase and the transglutaminase in combination.

### [Test Example 2]

Sucrose was added to 20 mL of soy milk so as to have a final concentration of 5 wt%, sterilized by autoclaving, cooled to 42°C, and kept warm. While maintaining the temperature at 42°C, 50 U of a laccase per 1 g of soybean protein was added and mixed for 5 seconds, and 20 U of a transglutaminase per 1 g of soybean protein was added and mixed for 5 seconds, yogurt inoculums (Lactococcus lactis subsp. Cremoris FC and Acetobacter orientalis FA) were added so that the total final concentration was 0.2 wt%, and mixed for 5 seconds, and the mixture was incubated at 42°C for 24 hours (crosslinking step associated with fermentation) and cooled to 4°C to prepare soy milk yogurt (Example 2). Soy milk yogurt (Comparative Example 2-1) prepared in the same manner except that neither laccase nor transglutaminase was used, soy milk yogurt (Comparative Example 2-2) prepared in the same manner using only a laccase between a laccase and a transglutaminase, and soy milk yogurt (Comparative Example 2-3) prepared in the same manner using only a transglutaminase between a laccase and a transglutaminase were prepared.

The strength (Firmness (N)) of the obtained soy milk yogurt was measured in the same manner as in Test Example 1.

As a result, as compared with the strength of the soy milk yogurt of Comparative Example 2-1 in which neither the laccase treatment nor the transglutaminase treatment was performed, the strength of the soy milk yogurt of Comparative Example 2-2 in which only the laccase treatment was performed was enhanced to 1.25 times, and the strength of the soy milk yogurt of Comparative Example 2-3 in which only the transglutaminase treatment was performed was enhanced to 1.33 times, whereas the strength of the soy milk yogurt of Example 2 in which both the laccase treatment and the transglutaminase treatment were performed was enhanced to 1.63 times. That is, in the soy milk yogurt of Example 2, an excellent strength enhancing effect exceeding the additive effect (effect of enhancing the strength to 1.58 times) of the strength enhancing effect exhibited by each of the laccase and the transglutaminase alone was observed.

## Claims

1. A method of producing a processed protein, comprising a crosslinking step of causing a laccase and a transglutaminase to act on a protein.

2. The method of producing a processed protein according to claim 1, wherein the protein is soybean protein.

3. The method of producing a processed protein according to claim 1 or 2, wherein the processed protein is tofu.

4. The method of producing a processed protein according to claim 1 or 2, wherein the processed protein is fermented soy milk.

5. The method of producing a processed protein according to any one of claims 1 to 4, wherein the laccase is used in an amount of 0.02 U or more per 1 g of the protein.

6. The method of producing a processed protein according to any one of claims 1 to 5, wherein the laccase is used in an amount of 30 U or more per 1 g of the protein.

7. The method of producing a processed protein according to any one of claims 1 to 6, wherein the transglutaminase is used in an amount of 0.02 U or more per 1 g of the protein.

8. The method of producing a processed protein according to any one of claims 1 to 7, wherein the transglutaminase is used in an amount of 0.0004 U or more per 1 U of the laccase.

9. The method of producing a processed protein according to any one of claims 1 to 8, wherein the laccase is derived from Trametes hirsuta.
